**Europäisches Patentamt**

**European Patent Office**

**Office européen des brevets**

(19)

(11) Numéro de publication : **0 384 804 B1**

(12) ## FASCICULE DE BREVET EUROPEEN

(45) Date de publication du fascicule du brevet :
**04.05.94 Bulletin 94/18**

(51) Int. Cl.⁵ : **A61K 7/48,** A61K 7/00,
A61N 1/30

(21) Numéro de dépôt : **90400379.5**

(22) Date de dépôt : **13.02.90**

(54) **Masque cutané conducteur de courant pour application thérapeutique ou esthétique.**

(30) Priorité : **15.02.89 FR 8901949**

(43) Date de publication de la demande :
**29.08.90 Bulletin 90/35**

(45) Mention de la délivrance du brevet :
**04.05.94 Bulletin 94/18**

(84) Etats contractants désignés :
**AT BE CH DE DK ES GB GR IT LI LU NL SE**

(56) Documents cités :
**EP-A- 0 004 514
EP-A- 0 159 167
DE-A- 1 589 524
FR-A- 2 047 874
FR-A- 2 503 561
US-A- 3 881 495**

(56) Documents cités :
**US-A- 4 210 633
CHEMICAL ABSTRACTS, vol. 91, 1979, page
332, résumé no. 216679e, Columbus, Ohio, US;
& JP-A-79 55 738 (KOBAYASHI KOSE CO.,
LTD) 04-05-1979
PARFUMS, COSMETIQUES, AROMES, no. 72,
décembre 1986, pages 61-64, Paris, FR; A.
JULIEN et al.: "Les masques de beauté"**

(73) Titulaire : **Ramond, Gérard
Gragnague
F-31380 Montastruc-La-Conseillere (FR)**

(72) Inventeur : **Ramond, Gérard
Gragnague
F-31380 Montastruc-La-Conseillere (FR)**

(74) Mandataire : **CABINET BONNET-THIRION
95 Boulevard Beaumarchais
F-75003 Paris (FR)**

## Description

L'invention se rapporte à un voile conducteur cutané, pour l'application à la peau d'un sujet de courants électriques dans un but thérapeutique ou esthétique, formé d'une couche de composition apte à faire prise, étalée sur la zone de peau, et présentant une conductivité substantielle au cours de la prise.

L'invention a trait également à un appareillage pour l'utilisation d'un tel voile, appliqué en masque sur le visage.

Le document de brevet EP-A-0 004 514 décrit un moulage électroconducteur formant électrode cutanée pour l'application sur des parties du corps humain de courants électriques à but thérapeutique ou esthétique. Le moulage, selon ce document, était constitué d'une poudre capable de prise avec l'eau, typiquement plâtre de qualité dentaire, et d'eau de prise en excès, contenant un sel dissous, typiquement chlorure de calcium. Des contacts métalliques sont enrobés dans le moulage, pour l'arrivée de courant depuis un générateur. On associe normalement une contre-électrode pouvant être un autre moulage. Comme l'achèvement de la prise rend le moulage pratiquement non conducteur, l'application de courant se fait entre le début et la fin de la prise de moulage.

A l'usage, il est apparu que la préparation des moulages électroconducteurs, leur mise en place sur les sujets, et le contrôle de l'application de courant de traitement thérapeutique ou esthétique demandaient à être faits par un personnel convenablement instruit et entraîné. De plus le sujet doit maintenir immobile la partie du corps où l'on applique le moulage tant que celui-ci n'a pas acquis une résistance suffisante.

Aussi les traitements thérapeutiques ou esthétiques de la peau par passage de courants électriques doivent être conduits dans des cliniques ou instituts spécialisés.

Il est ainsi apparu souhaitable, pour des traitements qui ne nécessitent pas une surveillance stricte par des professionnels, de mettre au point des électrodes cutanées susceptibles d'être appliquées et utilisées par le sujet lui-même.

Dans ce but, l'invention propose un voile conducteur cutané, à utiliser en association avec un générateur de courants pulsés, pour l'application à une zone de peau d'un sujet de tels courants dans un but thérapeutique ou esthétique, formé d'une couche de composition apte à faire prise, étalée sur la zone de peau, et présentant une conductivité substantielle au cours de la prise, caractérisé en ce que la composition est un gel polymérisant au contact de l'air, constitué d'un mélange ternaire alcool polyvinylique, éthanol et eau, avec un plastifiant physiologiquement acceptable sur la peau, et comporte, en poids, de 15 à 30 % d'alcool polyvinylique avec un taux d'hydrolyse supérieur à 85 %, de 7 à 15 % d'éthanol et, à titre de plastifiant, de 1,5 à 3 % de lanoline hydrosoluble et de 0,7 à 1,5 % de glycérol, le reste étant de l'eau, grâce à quoi, à l'achèvement de la polymérisation, l'application des courants à la zone de peau du sujet prend fin.

La polymérisation du gel ainsi constitué conduit à la formation d'un voile souple, qui est étroitement appliqué sur la peau et suit les déformations modérées de celle-ci pendant toute la polymérisation. Le voile présente une conductivité du même ordre que les tissus cutanés, jusqu'à achèvement de la polymérisation, dans des conditions comparables à celles des moulages électroconducteurs connus. Après achèvement de la polymérisation, le voile s'enlève, soit d'une pièce, soit par larges lambeaux, sans s'accrocher à la peau.

La composition la plus préférée comporte, en poids, sensiblement 20 % d'alcool polyvinylique, 10 % d'éthanol, 2 % de lanoline hydrosoluble, 1 % de glycérol, le reste étant de l'eau.

En disposition préférée, la quantité d'alcool polyvinylique est réglée, conjointement avec le choix du taux de condensation, pour une viscosité du gel comprise entre 2 000 et 3 000 Pa.s.

La nuance d'alcool polyvinylique préférée présente un indice d'ester de 70 % environ, et un taux de condensation tel que la viscosité de sa solution aqueuse à 4 % en poids soit de 3 Pa.s environ à 20°C.

Concurremment avec un gel capable de former un voile conducteur, que le sujet peut appliquer lui-même, il est apparu souhaitable que le générateur de courant de traitement soit rendu portable, ce qui autorise à mettre en oeuvre des traitements thérapeutiques et surtout esthétiques par le sujet lui-même et chez lui, chaque fois que le traitement n'appelle pas de surveillance particulière.

Sous un autre aspect, il est apparu que, pour traiter des zones cutanées importantes, il était préférable que les électrodes cutanées présentent un conductivité, parallèlement à la peau, qui corresponde à peu près à la conductivité des tissus sous-jacents. Lors de l'application d'une tension entre deux points distants d'un même voile, les gradients de tension sont analogues dans les tissus cutanés et les parties de voile qui les surmontent. On peut exécuter le traitement en reliant deux points éloignés d'un même voile aux deux bornes d'un générateur approprié. Il n'est dès lors pas nécessaire d'utiliser des contre-électrodes pour le retour de courant, ni de couper le voile par un intervalle pour constituer une paire d'électrodes. D'autant que la présence d'un intervalle entre deux demi-voiles a tendance à créer des gradients de tension relativement élevés dans la zone d'intervalle, et des courants localisés dans l'intervalle.

L'invention propose ainsi un appareillage pour le traitement cutané à but thérapeutique ou esthétique du visage d'un sujet, par passage dans les tissus superficiels d'un courant électrique pulsé, comportant un générateur de tension pulsée avec deux bornes de sortie, un voile conducteur appliqué en masque sur le visage, et des câbles de connexion des bornes de sortie du générateur au voile conducteur, caractérisé en ce que le voile conducteur est formé en masque d'une pièce sur le visage par application d'un gel polymérisant à l'air composé, en poids, de 15 à 30 % d'alcool polyvinylique avec un taux d'hydrolyse supérieur à 85 %, de 5 à 15 % d'éthanol, de 1,5 à 3 % de lanoline hydrosoluble, 0,7 à 1,5 % de glycérol, le reste étant de l'eau, et en ce que les câbles de liaison sont bifilaires et aboutissent chacun à une plaque conductrice aux deux extrémités, isolées, d'un arceau élastique propre à coiffer la tête du sujet, les plaques conductrices portant, par l'intermédiaire de tampons poreux et souples imprégnés d'une solution saline, sur le masque à l'arrière des joues du sujet, sensiblement à hauteur des oreilles.

Les éléments de cette disposition concourent tous à favoriser la mise en oeuvre individuelle des traitements de peau par des courants électriques.

Les caractéristiques et avantages de l'invention ressortiront d'ailleurs de la description qui va suivre, à titre d'exemple, en référence aux dessins annexés dans lesquels :

la figure 1 représente un voile conducteur selon l'invention, formant un masque pour le traitement électrique de la peau du visage, avec l'appareillage de traitement ;

la figure 2 est un détail de la prise de contact sur le masque en voile conducteur.

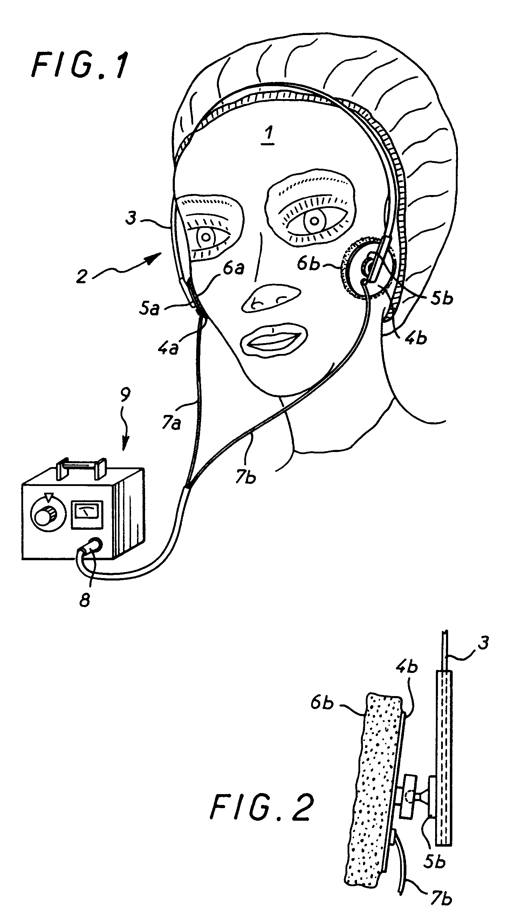

Selon la forme de réalisation choisie et représentée figure 1, un voile constituant un masque 1 a été formé sur le visage d'un sujet, depuis sensiblement la limite d'implantation des cheveux sur le front jusqu'à la base du cou, avec des réserves pour les yeux, les narines et la bouche. Ce masque 1 est obtenu à partir d'un gel capable de polymériser au contact de l'air quand il est étalé sur la peau, et qui est conducteur au cours de la polymérisation. La composition du gel sera précisée plus loin.

Sur le masque sont appliqués deux contacts 4a, 4b fixés aux extrémités d'un arceau élastique 3, avec interposition d'éléments isolants 5a, 5b respectivement. Les contacts 4a, 4b sont appuyés à l'arrière des joues sur le masque, avec interposition de tampons poreux 6a, 6b, rendus conducteurs par imprégnation d'une solution saline. Sur les contacts 4a, 4b sont fixés deux conducteurs respectifs 7a, 7b réunis en un cordon terminé par une fiche bifilaire 8, qui s'engage dans un jack d'un générateur de courant pulsé 9. On remarquera que l'ensemble 2 de l'arceau 3, des contacts 4a, 4b et du cordon présente une ressemblance avec un casque d'écoute, tel par exemple un casque de "baladeur".

Comme on le voit mieux à la figure 2, l'extrémité de l'arceau 3 est gainée d'isolant, dont fait saillie une rotule isolante 5b, qui s'engage dans un logement complémentaire sur lequel est fixée une plaque métallique circulaire 4b, d'où part le conducteur 7b. Sur la plaque de contact 4b vient s'appliquer un tampon ou éponge 6b. Bien entendu, à l'autre extrémité de l'arceau 3, on trouve une rotule isolante 5a, une plaque de contact 4a d'où part le conducteur 7a, et un tampon éponge 7a.

Le générateur 9 délivre des tensions pulsées, à tension de crête et fréquence de récurrence réglables, telles qu'on les utilise pour des traitements thérapeutiques ou esthétiques. La forme et la polarité de ces impulsions ne seront pas détaillées ici, car elles sont étrangères à l'invention. On indiquera que la tension de crête peut atteindre 150 volts, et que la fréquence de récurrence est comprise entre 40 et 110 Hz.

Le courant efficace maximal que peut délivrer le générateur 9 se situe vers 2 mA, cette intensité correspondant à un traitement intéressant une surface importante de peau, telle que celle du buste. Ce générateur 9 peut, en raison de la puissance réduite qu'on lui demande, être autonome et fonctionner sur batterie 9 volts.

On aura remarqué que les contacts 4a et 4b sont appliqués sur le masque qui forme une couche continue ; ainsi une tension appliquée entre ces contacts 5a, 5b va faire passer un courant directement dans le masque lui-même, et un courant dans les tissus cutanés sous-jacents. Bien que le courant qui passe dans le masque corresponde à une perte d'énergie, il établit un gradient de potentiel entre les contacts 4a, 4b. Si les résistivités du masque et des tissus sous-jacents sont du même ordre de grandeur, les courants dérivés dans le masque restent d'importance limitée, mais la répartition des potentiels atténue l'incidence de zones de conductivité anormale des tissus cutanés.

Typiquement la composition pondérale du gel utilisé pour le masque est la suivante :

```
Alcool polyvinylique Rhodoviol 30/70 ®        20 %

Ethanol                                       10 %

Glycérol                                       1 %

Lanoline hydrosoluble                          2 %

Eau désionisée                Complément à 100 %
```

Le mélange présente une viscosité à 20°C de 2 490 Pascal.seconde (24 900 centipoises) environ, et une résistivité volumique de 27,5 ohm.mètre.

Ce mélange, conservé en masse à l'abri de l'air, est de caractéristiques assez stables, et n'exige pas une préparation extemporanée.

Par contre, appliqué en couche sur la peau, le gel prend consistance très rapidement ; c'est alors qu'il est utilisable en voile conducteur. Après une durée d'une demi-heure environ, la polymérisation est pratiquement complète et la résistance électrique devient très élevée. Le voile, notamment le masque, s'enlève alors d'une pièce, mais est toujours souple.

La composition du gel reste utilisable avec des écarts de composition de l'ordre de $\pm$ 50 %, étant entendu que la quantité d'eau est toujours le complément à 100 %.

On notera que la vitesse de polymérisation s'ajuste par la teneur en éthanol.

On a effectué divers essais avec des alcools polyvinyliques Rhodoviol® de Rhône-Poulenc, essentiellement :

Rhodoviol     30/70
Rhodoviol     4/125
Rhodoviol     25/140

Le premier nombre rend compte de l'état de condensation de l'alcool polyvinylique, exprimé par la viscosité en centipoises (1 CP = 10 Pa.s) de la solution à 4 % en poids dans l'eau à 20°C. Le second nombre rend compte du taux d'hydrolyse, exprimé en indice d'ester conventionnel. La correspondance entre l'indice d'ester et le taux d'hydrolyse (en molécules pour cent) est donnée dans le tableau suivant :

| Indice ester | Taux d'hydrolyse (mol %) |
|:---:|:---:|
| 70 | 94 % |
| 125 | 89 % |
| 140 | 88 % |

Les meilleurs résultats sont obtenus pour des taux d'hydrolyse supérieurs à 90 %, et des viscosités en solution à 4 % supérieure à 2 Pa.s. La viscosité du gel préparé sera de préférence comprise entre 2 000 et 3 000 Pa.s, pour permettre un étalement aisé sans que l'épaisseur du voile soit trop faible.

## Revendications

1. Voile conducteur cutané, à utiliser en association avec un générateur de courants pulsés, pour l'application à une zone de peau d'un sujet de tels courants dans un but thérapeutique ou esthétique, formé d'une couche de composition apte à faire prise, étalée sur la zone de peau, et présentant une conductivité substantielle au cours de la prise, caractérisé en ce que la composition est un gel polymérisant au contact de l'air, constitué d'un mélange ternaire alcool polyvinylique, éthanol et eau, avec un plastifiant physiologiquement acceptable sur la peau, et comporte, en poids, de 15 à 30 % d'alcool polyvinylique avec un taux d'hydrolyse supérieur à 85 %, de 7 à 15 % d'éthanol et, à titre de plastifiant, de 1,5 à 3 % de lanoline hydrosoluble et de 0,7 à 1,5 % de glycérol, le reste étant de l'eau, grâce à quoi, à l'achèvement de la polymérisation, l'application des courants à la zone de peau du sujet prend fin.

2. Voile conducteur selon la revendication 1, caractérisé en ce que la composition comporte, en poids, sensiblement 20 % d'alcool polyvinylique, 10 % d'éthanol, 2 % de lanoline hydrosoluble, 1 % de glycérol, le reste étant de l'eau.

3. Voile conducteur selon l'une des revendications 1 et 2, caractérisé en ce que la quantité d'alcool polyvinylique dans la composition est réglée, conjointement avec le choix d'un taux de condensation, pour une viscosité du gel comprise entre 2 000 et 3 000 Pa.s.

4. Voile conducteur selon l'une des revendications 1 et 2, caractérisé en ce que l'alcool polyvinylique présente un indice d'ester de 70 environ, et un taux de condensation tel que la viscosité de sa solution aqueuse à 4 % en poids soit de 3 Pa.s environ à 20°C.

5. Appareillage pour le traitement cutané à but thérapeutique ou esthétique du visage d'un sujet, par passage dans les tissus superficiels d'un courant électrique pulsé, comportant un générateur (9) de tension pulsée avec deux bornes de sortie, un voile conducteur (1) applicable en masque sur le visage, et des câbles de connexion (7a, 7b) des bornes de sortie du générateur au voile conducteur, caractérisé en ce que le voile conducteur est apte de formé en masque d'une pièce sur le visage par application d'un gel polymérisant à l'air composé, en poids, de 15 à 30 % d'alcool polyvinylique avec un taux d'hydrolyse supérieur à 85 %, de 5 à 15 % d'éthanol, de 1,5 à 3 % de lanoline hydrosoluble, 0,7 à 1,5 % de glycérol, le reste étant de l'eau, et en ce que les câbles (7a, 7b) de liaison sont bifilaires et aboutissent chacun à une plaque conductrice (4a, 4b) aux deux extrémités, isolées, d'un arceau élastique (3) propre à coiffer la tête du sujet, les plaques conductrices (4a, 4b) portant, par l'intermédiaire de tampons poreux (6a, 6b) et souples imprégnés d'une solution saline, sur le masque (1) à l'arrière des joues du sujet, sensiblement à hauteur des oreilles.

6.  Appareillage selon la revendication 5, caractérisé en ce que le générateur (9) possède une alimentation autonome.

7.  Appareillage selon l'une des revendications 5 et 6, caractérisé en ce que le générateur (9) est adapté à délivrer des impulsions avec une tension de crête atteignant 150 V, une intensité efficace jusqu'à 2 mA, et une fréquence de récurrence comprise entre 40 et 110 Hz.

**Patentansprüche**

1.  Leitende Abdeckung für die Haut zur Verwendung in Verbindung mit einem Generator für pulsierende Ströme, um solche Ströme für einen therapeutischen oder ästhetischen Zweck auf einer Hautzone einer Person anzulegen, die aus einer Schicht eines Materials gebildet ist, das befähigt ist, fest zu werden, auf der Hautzone aufgetragen ist und eine beträchtliche Leitfähigkeit im Verlauf der Verfestigung aufweist, dadurch gekennzeichnet, daß das Material ein Gel ist, das beim Kontakt mit Luft polymerisiert, aus einer ternären Mischung von Polyvinylalkohol, Ethanol und Wasser mit einem auf der Haut physiologisch akzeptablen Weichmacher gebildet ist und, in Gewichtsprozenten, 15 bis 30 % Polyvinylalkohol mit einem Hydrolysegrad von über 85 %, 7 bis 15 % Ethanol und als Weichmacher 1,5 bis 3 % wasserlösliches Lanolin und 0,7 bis 1,5 % Glycerin sowie als Rest Wasser enthält, wodurch bei Beendigung der Polymerisation die Anlegung der Ströme auf der Hautzone der Person zu Ende geht.

2.  Leitende Abdeckung nach Anspruch 1, dadurch gekennzeichnet, daß das Material, in Gewichtsprozenten, etwa 20 % Polyvinylalkohol, 10 % Ethanol, 2 % wasserlösliches Lanolin, 1 % Glycerin und als Rest Wasser enthält.

3.  Leitende Abdeckung nach einem der Ansprüche 1 und 2, dadurch gekennzeichnet, daß die Menge des Polyvinylalkohols in dem Material zusammen mit der Wahl eines Kondensationgrades für eine Viskosität des Gels im Bereich zwischen 2000 und 3000 Pa·s eingestellt ist.

4.  Leitende Abdeckung nach einem der Ansprüche 1 und 2, dadurch gekennzeichnet, daß der Polyvinylalkohol einen Esterindex von ungefähr 70 und einen solchen Kondensationsgrad aufweist, daß die Viskosität seiner 4 Gew.-% igen wäßrigen Lösung ungefähr 3 Pa·s bei 20°C beträgt.

5.  Vorrichtung für die Hautbehandlung des Gesichts einer Person zu therapeutischem oder ästhetischem Zweck, indem man einen pulsierenden elektrischen Strom in die Hautgewebe einleitet, mit einem Generator (9) für pulsierende Spannung mit zwei Ausgangsklemmen, einer leitenden Abdeckung (1), die als Maske auf das Gesicht auftragbar ist, und Verbindungskabeln (7a, 7b) von den Ausgangsklemmen des Generators zur leitenden Abdeckung, dadurch gekennzeichnet, daß die leitende Abdeckung geeignet ist, als Maske aus einem Stück auf dem Gesicht gebildet zu werden durch Auftrag eines Gels, das an Luft polymerisiert, und zusammengesetzt ist, in Gewichtsprozenten, aus 15 bis 30 % Polyvinylalkohol mit einem Hydrolysegrad von über 85 %, 5 bis 15 % Ethanol, 1,5 bis 3 % wasserlöslichem Lanolin, 0,7 bis 1,5 % Glycerin, wobei der Rest Wasser ist, und daß die Verbindungskabel (7a, 7b) zweiadrig sind und jedes an einer leitenden Platte (4a, 4b) an den zwei isolierten äußersten Enden eines elastischen Bügels (3) endet, der geeignet ist, den Kopf der Person zu bedecken, wobei die leitenden Platten (4a, 4b) durch Zwischenschalten von porösen und nachgiebigen Tampons (6a, 6b), die mit einer Salzlösung getränkt sind, auf der Maske (1) hinter den Wangen der Person etwa auf der Höhe der Ohren liegen.

**6.** Vorrichtung nach Anspruch 5, dadurch gekennzeichnet, daß der Generator (9) eine unabhängige Stromversorgung hat.

**7.** Vorrichtung nach einem der Ansprüche 5 und 6, dadurch gekennzeichnet, daß der Generator (9) ausgelegt ist, Impulse mit einer Spitzenspannung, die 150 V erreichen, eine wirksame Intensität bis zu 2 mA und eine Wiederholfrequenz im Bereich von 40 und 110 Hz zu liefern.

## Claims

**1.** A conductive skin mask to be used in association with a generator for generating pulsed currents, for the application of such currents to a region of the skin of a subject for therapeutic or aesthetic purposes, which is formed by a layer of a composition capable of setting, when spread over the region of skin, and being of substantial conductivity in the course of setting, characterised in that the composition is a gel which polymerises in contact with the air, formed by a polyvinyl alcohol, ethanol and water ternary mixture, with a plasticiser which is physiologically acceptable on the skin, and comprises by weight from 15 to 30% of polyvinyl alcohol with a hydrolysis factor of higher than 85%, from 7 to 15% of ethanol and, as plasticiser, from 1.5 to 3% of water-soluble lanolin and from 0.7 to 1.5% of glycerol, the balance being water, whereby upon conclusion of polymerisation, application of the currents to the region of the skin of the subject comes to an end.

**2.** A conductive mask according to claim 1 characterised in that the composition comprises by weight substantially 20% of polyvinyl alcohol, 10% of ethanol, 2% of water-soluble lanolin and 1% of glycerol, the balance being water.

**3.** A conductive mask according to one of claims 1 and 2 characterised in that the amount of polyvinyl alcohol in the composition is regulated jointly with the choice of a condensation rate, for a viscosity of the gel of between 2000 and 3000 Pa.s.

**4.** A conductive mask according to one of claims 1 and 2 characterised in that the polyvinyl alcohol has an ester value of about 70 and a condensation rate such that the viscosity of its aqueous 4% by weight solution is 3 Pa.s approximately at 20°C.

**5.** Apparatus for skin treatment for therapeutic or aesthetic purposes of the face of a subject, by passing a pulsed electrical current into the surface tissues, comprising a pulsed voltage generator (9) with two output terminals, a conductive mask (1) which can be applied to the face to mask same, and cables (7a, 7b) for connecting the output terminals of the generator to the conductive mask characterised in that the conductive mask is capable of being formed as a one-piece mask on the face by the application of a gel which polymerises in the air and which is composed by weight of from 15 to 30% of polyvinyl alcohol with a hydrolysis factor of higher than 85%, from 5 to 15% of ethanol, from 1.5 to 3% of water-soluble lanolin, and from 0.7 to 1.5% of glycerol, the balance being water, and that the connecting cables (7a, 7b) are two-wire cables and each terminate at a conductive plate (4a, 4b) at the two insulated ends of a resilient loop (3) for fitting over the head of the subject, the conductive plates (4a, 4b) bearing by way of porous flexible pads (6a, 6b) impregnated with a saline solution against the mask (1) rearwardly of the cheeks of the subject substantially at the level of the ears.

**6.** Apparatus according to claim 5 characterised in that the generator (1) has a self-contained supply.

**7.** Apparatus according to one of claims 5 and 6 characterised in that the generator (9) is adapted to deliver pulses with a peak voltage attaining 150 V, an effective current strength of up to 2mA and a repetition rate of between 40 and 110 Hz.

FIG.1

FIG.2